# EUROPÄISCHE PATENTANMELDUNG

(11) **EP 1 088 809 A1**
(43) Veröffentlichungstag der Anmeldung: **04.04.2001**
(21) Anmeldenummer: 00117403.6
(22) Anmeldetag: 11.08.2000
(51) Int. Cl.: C07C 37/08, C07C 45/53

(54) **Verfahren zur Herstellung von Phenol, Aceton und Methylethylketon**

(30) Priorität: 30.09.1999 DE 19946888
(71) Anmelder: Phenolchemie GmbH & Co. KG, 45966 Gladbeck (DE)
(72) Erfinder: Pompetzki, Werner, Dr., 46284 Dorsten (DE); Gerlich, Otto, Dr., 45966 Gladbech (DE); Kleinloh, Werner, Dr., 45721 Haltern (DE)
(74) Vertreter: Olbricht, Gerhard, Dr.

(57) **Zusammenfassung**

Phenol wird überwiegend durch Hocksche Spaltung von Cumolhydroperoxid hergestellt, wobei als Koppelprodukt zwangsläufig zu gleichen molaren Teilen Aceton anfällt, für welches in Zukunft aber geringere Absatzmärkte als für Phenol prognostiziert werden.

Erfindungsgemäß werden Phenol und Aceton daher durch Oxidation eines Cumol und bis zu 25 Gew.-% sek.-Butylbenzol enthaltenden Gemischs und anschließende Hocksche Spaltung der Hydroperoxide hergestellt, so daß über die Zusammensetzung des Eduktgemisches das Verhältnis der Wertprodukte Phenol:Aceton:Methylethylketon gesteuert werden kann.

Herstellung von Phenol, Aceton und Methylethylketon.

## Beschreibung

Die Erfindung betrifft ein Verfahren zur Herstellung von Phenol, Aceton und Methylethylketon durch Oxidation eines Cumol und sek.-Butylbenzol enthaltenden Gemisches und anschließende Hocksche Spaltung von Cumolhydroperoxid und sek.-Butylbenzolhydroperoxid.

Phenol ist ein wichtiger chemischer Grundstoff mit breiter Anwendungspalette. Neben seiner Verwendung als Lösemittel wird Phenol unter anderem zur Herstellung von Phenol-Harzen, Bisphenol-A, ε-Caprolactam, Adipinsäure, Alkylphenolen und Weichmachern eingesetzt.

Es ist bekannt, Phenol durch Hocksche Spaltung eines geeigneten Hydroperoxids herzustellen. Dabei entsteht neben dem Phenol als Hydroxy-Verbindung stets als Koppelprodukt eine Carbonyl-Verbindung, die aus wirtschaftlichen Gründen ebenfalls einer geeigneten Verwertung zugeführt werden muß.

Die Offenlegungsschrift EP-0 548 986 A1 lehrt ein Verfahren zur Herstellung von Phenol und Methylethylketon (kurz: MEK) als Koppelprodukt durch Oxidation von sek.-Butylbenzol zu sek.-Butylbenzolhydroperoxid und Hocksche Spaltung des sek. - Butylbenzolhydroperoxids zu Phenol und MEK. MEK ist neben Aceton eines der technisch wichtigsten Ketone, das vor allem als Lack- und Harzlösemittel verwendet wird. In EP-0 548 986 A1 wird vorgeschlagen, als Ausgangsmaterial ein sek.-Butylbenzol einzusetzen, das entweder im wesentlichen frei von Ethylhydroperoxid, Carbonsäuren und Phenol oder im wesentlichen frei von styrolischen Verbindungen oder im wesentlichen frei von Methylbenzylalkohol ist. Durch geeignete, auf diese Einsatzmaterialien abgestimmte Prozeßführungen mit speziellen zusätzlichen Behandlungsschritten wird vor allem eine verbesserte Abtrennung unerwünschter Nebenprodukte erzielt. Dadurch kann nicht umgesetztes sek.-Butylbenzol zum Oxidationsschritt rückgeführt werden, ohne die Oxidationsgeschwindigkeit negativ zu beeinflussen.
Auch die Offenlegungsschrift EP-0 578 194 A2 betrifft ein Verfahren zur Herstellung von Phenol und MEK aus sek.-Butylbenzol. Es wird ein Verfahren beschrieben, das im Anschluß an die üblichen Verfahrensschritte Oxidation, Aufkonzentrierung und Spaltung spezielle Aufbereitungsschritte, insbesondere eine Alkaliwäsche der durch Destillation abgetrennten MEK-Fraktion, aufweist, wodurch insgesamt ein MEK höherer Reinheit erhalten werden soll.

Aus dem US-Patent 4 532 360 ist ein direktes, einstufiges Verfahren bekannt, das auch zur Herstellung von Phenol und MEK aus sek.-Butylbenzol verwendet werden kann. Dabei wird die Oxidation von sek.-Butylbenzol in Gegenwart von Bromwasserstoff oder Chlorwasserstoff sowie mindestens eines Additivs aus der Gruppe Ceriumoxid, Triphenylborat, Bortriphosphat und Wasser durchgeführt, wodurch eine direkte Spaltung des sek.-Butylbenzolhydroperoxids zu Phenol und MEK eintritt.

Aus dem Patentdokument JP-62-114 922 vom 26. Mai 1987 ist ein Verfahren zur gleichzeitigen Herstellung von Phenol, MEK und Aceton durch Oxidation von sek.-Butylbenzol mit molekularen Sauerstoff enthaltendem Gas in Gegenwart von Cumol oder Cumolhydroperoxid bekannt. Durch den Zusatz zum sek.-Butylbenzol von 5 bis 60 Gew.-% eines Cumolhydroperoxidkonzentrats, das 65 bis 85 Gew.-% Cumolhydroperoxid enthält, oder durch Zugabe von mindestens 50 Gew.-%, idealerweise jedoch 30 bis 70 Gew.-%, Cumol zum sek.-Butylbenzol, wobei sich die Angabe der Gew.-% auf dem Gehalt an sek.-Butylben zol bezieht, wird eine erhöhte Reaktionsgeschwindigkeit bei der Oxidation des sek.-Butylbenzols erzielt. Ferner wird die Abhängigkeit der Oxidationsgeschwindigkeit des sek.-Butylbenzols vom Isobutylbenzolgehalt und die Bildung des Nebenprodukts Acetophenon verringert. Im Falle des Zusatzes von Cumol zum sek.-Butylbenzol wird also mit einem Unterschuß an Cumol oxidiert.

Phenol wird heutzutage allerdings in der Hauptsache durch Hocksche Spaltung von Cumolhydroperoxid hergestellt, wobei als Koppelprodukt Aceton anfällt. Aceton besitzt ebenfalls vielfältige Anwendungsmöglichkeiten, nämlich z. B. als Lösemittel oder zur Herstellung von u. a. Bisphenol-A und Methylmethacrylat.

Bei diesem sogenannten Cumolverfahren zur Herstellung von Phenol und Acteon wird zunächst Cumol mit vorzugsweise Luft oder Sauerstoff zum Cumolhydroperoxid oxidiert, welches - nach üblicherweise einer destillativen Aufkonzentrierung durch Abtrennung von nicht umgesetztem Cumol auf einen Cumolhydroperoxidgehalt von 60 bis 85 Gew.-% - anschließend unter Säurekatalyse mit vorzugsweise Schwefelsäure zu Phenol und Aceton gespalten wird. Einen guten Überblick über das Cumolverfahren geben z. B. Weissermel/Arpe, Industrielle Organische Chemie, 2. Auflage, Verlag Chemie, 1978, oder Ullmann's Encyclopedia of Industrial Chemistry, Vol. A 19, S. 302 ff, VCH Verlagsgesellschaft, 1991. Neuere Weiterentwicklungen des Cumolverfahrens betreffen vor allem den Bereich der Cumolhydroperoxid-Spaltung und der Aufarbeitung des Spaltproduktes zur Verringerung der Nebenproduktbildung und des Energieverbrauchs, vgl. z. B. EP-0 589 588 A1, EP-0 670 296 A1 oder WO 97/06905.

Das Cumolverfahren führt stets molar zu gleichen Teilen zur Gewinnung von Phenol und Aceton. Bezogen auf das Gewicht fallen pro Tonne Phenol 0,62 Tonnen Aceton an. Ein wesentlicher Verwendungszweck für beide Produkte ist die Bisphenol-A-Synthese, wobei entsprechend der Reaktionsgleichung ausgehend von 2 mol Phenol und 1 mol Aceton 1 mol Bisphenol-A erhalten wird. Bisphenol-A dient als -Ausgangsstoff zur Herstellung von Polycarbonaten und Epoxidharzen und wird daher in großen Mengen produziert. Zur Synthese von Bisphenol-A wird aber - molar betrachtet - nur halb so viel Aceton wie Phenol benötigt. Zwar besitzt Aceton wie erwähnt auch weitere Verwendungsmöglichkeiten, z. B. als Lösemittel oder zur Herstellung von z. B. Methylmethacrylat, doch wird insgesamt ein unterschiedliches Marktwachstum für Phenol und Aceton in der Weise prognostiziert, daß molar mehr Phenol als Aceton benötigt werden wird. Diesem unterschiedlichen Marktbedürfnis vermag das für die Herstellung von Phenol und Aceton hauptsächlich verwendete Cumolverfahren aufgrund seines Reaktionsschemas jedoch nicht zu entsprechen.

Damit stellt sich die Aufgabe, ein Verfahren zur Herstellung von Phenol bereitzustellen, das zwar ebenfalls Aceton produziert, aber anders als das Cumolverfahren nicht zwangsläufig äquimolare Mengen an Phenol und Aceton erzeugt, sondern auf wirtschaftliche Weise eine flexible Anpassung der Produktion an eine - molar betrachtet - voraussichtlich geringfügig höhere Phenol- als Acetonnachfrage erlaubt.

Diese Aufgabe wird erfindungsgemäß gelöst gemäß Patentanspruch 1 durch ein Verfahren zur Herstellung von Phenol, Aceton und Methylethylketon durch Oxidation eines Cumol und sek.-Butylbenzol enthaltenden Gemisches mit molekularen Sauerstoff enthaltendem Gas und anschließende Hocksche Spaltung von Cumolhydroperoxid und sek.-Butylbenzolhydroperoxid, das dadurch gekennzeichnet ist, daß das Cumol und sek.-Butylbenzol enthaltende Gemisch bis zu 25 Gew.-% sek.-Butylbenzol enthält. Vorzugsweise besteht das Gemisch nur aus Cumol und sek.-Butylbenzol.

Überraschenderweise wurde gefunden, daß mit Hilfe der aus dem Cumolverfahren bekannten Verfahrensschritte auch Gemische, die gleichzeitig Cumol und geringe Mengen von bis zu 25 Gew.-% an sek.-Butylbenzol enthalten, behandelt werden und gemeinsam zu Phenol, Aceton und MEK umgesetzt werden können. Damit kann durch Anpassung der Zusammensetzung des Eduktgemisches das molare Verhältnis von Phenol:Aceton:MEK im Produktgemisch gezielt gesteuert werden und in der Regel ein nicht zu vermarktender Überschuß an einer der beiden entstehenden Carbonylverbindungen vermieden werden. Das erfindungsgemäße Verfahren bietet damit den wesentlichen Vorteil, die Produktion an die Märkte für die Wertprodukte Phenol, Aceton und MEK anpassen zu können, wobei insbesondere die prognostizierte Aceton-Überkapazität vermieden werden kann.

Da die erfindungsgemäße Umsetzung des Cumol und bis zu 25 Gew.-% sek.-Butylbenzol bezogen auf die Mischung enthaltenden Gemischs unter den aus dem herkömmlichen Cumolverfahren bekannten Bedingungen erfolgen kann, ist das erfindungsgemäße Verfahren zudem schnell und einfach in bestehenden nach dem Cumolverfahren arbeitenden Anlagen realisierbar. Vorzugsweise wird bei der Realisierung im Bereich der Oxidation und der Spaltung auf bestehende Anlagenteile zurückgegriffen; lediglich bei der Zuführung der Edukte und insbesondere bei der Aufarbeitung des Spaltproduktstroms zu den einzelnen Wertprodukten sind Anpassungen erforderlich. Eine bestehende, nach dem herkömmlichen Cumolverfahren arbeitende Produktionsanlage für Phenol und Aceton kann daher leicht so erweitert werden, daß sie zur Durchführung des erfindungsgemäßen Verfahrens geeignet ist, und je nach Marktsituation erfindungsgemäß oder nur zur Herstellung von Phenol und Aceton betrieben werden.

Als Edukt für die Oxidation beim erfindungsgemäßen Verfahren dient also ein Gemisch, das neben Cumol erfindungsgemäß bis zu 25 Gew.-%, vorzugsweise bis 20 Gew.-%, besonders bevorzugt 10 bis 20 Gew.-%, sek.-Butylbenzol aufweist. Dieses Gemisch kann bereits in Lagertanks vorgehalten werden oder durch Vermischen aus getrennten Quellen erst vor dem Eintritt in die Oxidationsreaktoren gebildet werden. Es ist auch möglich, Cumol oder sek.-Butylbenzol enthaltende Stoffströme getrennt in die Oxidationsreaktoren einzudosieren und dort zu vermischen.

Vorzugsweise wird das erfindungsgemäße Eduktgemisch für die Oxidation direkt durch Alkylierung von Benzol mit einem entsprechenden Gemisch aus Propen und Buten-1/Buten-2 in Gegenwart eines handelsüblichen Alkylierungskatalysators wie z. B. AlCl₃, H₃PO₄/SiO₂ oder Zeolithen hergestellt. Das Propen, Buten-1 und Buten-2 enthaltende Gasgemisch kann dabei direkt von einem Cracker bezogen werden, wodurch eine zusätzliche Auftrennung des Gasgemisches in Propen und Buten-1/Buten-2 für z. B. eine getrennte Herstellung von Cumol und sek.-Butylbenzol entfallen kann.

Als Oxidationsreaktoren dienen vorzugsweise die vom Cumolverfahren bekannten Blasensäulenreaktoren. Die Oxidation erfolgt analog zum Cumolverfahren vorzugsweise ohne Katalysator bei Temperaturen von 100 °C bis 140 °C und Drücken von 1 bis 20 bar absolut in Gegenwart von bevorzugt Luft oder Sauerstoff als molekularen Sauerstoff enthaltendem Gas, bis ein Gehalt von bis zu 40 Gew-% an Peroxiden im Produktstrom aus der Oxidation erreicht ist. Der Rest umfaßt überwiegend nicht umgesetztes Cumol und sek.-Butylbenzol, da vorzugsweise ein weitestgehend reines Cumol/sek.-Butylbenzol-Gemisch eingesetzt wird. Ferner sind in geringen Mengen einige bei der Oxidation gebildete Nebenprodukte enthalten. Hierzu zahlen insbesondere Dimethylphenylcarbinol, 2-Phenylbutanol, Acetophenon und Propiophenon.

Wie beim herkömmlichen Cumolverfahren kann der Produktstrom aus der Oxidation direkt oder über eine Vorlage als Zwischenspeicher einer Konzentrierungseinheit zugeführt werden, in der vorzugsweise destillativ unter Vakuum durch Abtrennung von nicht umgesetztem Cumol und/oder sek.-Butylbenzol der Gehalt an Cumolhydroperoxid bevorzugt auf 40 bis 65 Gew.-% und/oder an sek.-Butylbenzolhydroperoxid auf bevorzugt 5 bis 15 Gew.-% im Stoffstrom erhöht wird. Das abgetrennte Cumol und/oder sek.-Butylbenzol wird vorzugsweise ggf. nach weiterer Aufbereitung zur Oxidation zurückgeführt.

Der Stoffstrom aus der Aufkonzentrierung wird nun der Spaltung zugeführt, die erfindungsgemäß bevorzugt säurekatalsiert, vorzugsweise mittels Schwefelsäure, in homogener Phase erfolgt. Cumolhydroperoxid und sek.-Butylbenzolhydroperoxid werden hier im wesentlichen zu Phenol, Aceton und MEK umgesetzt. Geeignete Spaltreaktoren und Reaktionsbedingungen sind z. B. aus EP-0 589 588 A1 oder WO 97/06905 bekannt. Es kann vorteilhaft sein, das Spaltprodukt analog zum Verfahren in diesen Patentdokumenten einer Nachtemperung zur Verringerung des Gehalts an unerwünschten, die Ausbeute verringenden Nebenprodukten zu unterziehen. Anschließend erfolgt eine vorzugsweise destillative Aufarbeitung des Spaltproduktgemisches auf dem Fachmann vertraute, zum Cumolverfahren analoge Weise, um die Wertprodukte Phenol, Aceton und MEK zu isolieren.

Das erfindungsgemäße Verfahren ist nicht auf die hier skizzierten Hauptverfahrensschritte beschrankt; vielmehr können alle aus dem Cumolverfahren bekannten Verfahrensvarianten, Ergänzungen oder Verschaltungen, insbesondere wenn sie darauf abzielen, die Nebenproduktbildung zu verringern und den Energieverbrauch zu optimieren, auch auf das erfindungsgemäße Verfahren übertragen werden. In Kenntnis des erfindungsgemäßen Verfahrens erschließen sich dem Fachmann vor dem Hintergrund des Cumolverfahrens vielfältige verfahrenstechnische und apparative Ausgestaltungen.

Mit Hilfe des erfindungsgemäßen Verfahrens lassen sich Spaltausbeuten an Phenol, Aceton und MEK von über 90 % erreichen. Die Zusammensetzung des in der Oxidation eingesetzten Eduktgemisches wird dabei hinsichtlich ihres Gehalts an sek.-Butylbenzol bevorzugt derart angepaßt, daß für die Wertprodukte Aceton und MEK, die als Koppelprodukte zum Phenol anfallen, jeweils ausreichende Absatzmöglichkeiten bestehen. Auf diese Weise werden Verluste durch Überkapazitäten vermieden und eine hohe Wirtschaftlichkeit erreicht.

Das erfindungsgemäße Verfahren wird durch das folgende Beispiel näher erläutert, ohne darauf beschränkt zu sein:

### Beispiel 1:

Ein Gemisch, bestehend aus 80 Gew.-% Cumol und 20 Gew.-% sek.-Butylbenzol, wird bei 132 °C mit Sauerstoff in einem thermostatisierbaren Blasensäulenreaktor oxidiert. Nach einer Oxidationszeit von 2,5 Stunden enthält das Oxidat laut Analyse mit einem Gaschromatograph (GC-Analyse) 21,5 Gew.-% Cumolhydroperoxid und 2,9 Gew.-% sek.-Butylbenzolhydroperoxid. Dieses Gemisch wird anschließend im Hochvakuum einer Konzentrierung unterworfen, wobei das anfallende Konzentrat laut Gaschromatograph-Analyse 59,3 Gew.-% Cumolhydroperoxid und 8,1 Gew.-% sek.-Butylbenzolhydroperoxid enthält. Das als Destillat anfallende Kohlenwasserstoffgemisch enthält weniger als 1 % Peroxid, berechnet als Cumolhydroperoxid. Das Konzentrat wird anschließend bei 50 °C einer einphasigen Spaltung in Gegenwart von 2000 ppm Schwefelsäure, gelöst in Aceton oder in einem Testspaltprodukt bestehend aus Aceton, MEK, Phenol und den Kohlenwasserstoffen gespalten.

Die Spaltausbeuten betragen nach Auswertung der GC-Analyse für Aceton über 95 %, für MEK 92,3 % und für Phenol über 99 %.

## Patentansprüche

1. Verfahren zur Herstellung von Phenol, Aceton und Methylethylketon durch Oxidation eines Cumol und sek.-Butylbenzol enthaltenden Gemisches mit molekularen Sauerstoff enthaltendem Gas und anschließende Hocksche Spaltung von Cumolhydroperoxid und sek.-Butylbenzolhydroperoxid,
dadurch gekennzeichnet,
daß das Cumol und sek. -Butylbenzol enthaltende Gemisch bis zu 25 Gew.-% sek.-Butylbenzol enthält.

2. Verfahren nach Anspruch 1,
dadurch gekennzeichnet,
daß das Gemisch bis zu 20 Gew.-% sek.-Butylbenzol enthält.

3. Verfahren nach Anspruch 2,
dadurch gekennzeichnet,
daß das Gemisch 10 bis 20 Gew.-% sek.-Butylbenzol enthält.

4. Verfahren nach zumindest einem der vorherigen Ansprüche,
dadurch gekennzeichnet,
daß das Gemisch sich aus Cumol und sek.-Butylbenzol jeweils hoher Reinheit zusammensetzt.

5. Verfahren nach zumindest einem der vorherigen Ansprüche,
dadurch gekennzeichnet,
daß das Gemisch durch Alkylierung von Benzol mit einem Propen, Buten-1 und Buten-2 enthaltenden Gasgemisch erzeugt wird.

6. Verfahren nach zumindest einem der vorherigen Ansprüche,
dadurch gekennzeichnet,
daß die Oxidation mit Luft oder Sauerstoff als molekularen Sauerstoff enthaltendem Gas erfolgt.

7. Verfahren nach zumindest einem der vorherigen Ansprüche,
dadurch gekennzeichnet,
daß die Oxidation bei Temperaturen von 100 °C bis 140 °C erfolgt.

8. Verfahren nach zumindest einem der vorherigen Ansprüche,
dadurch gekennzeichnet,
daß das Oxidat vor der Spaltung destillativ durch Abtrennung von Cumol oder sek.-Butylbenzol oder beidem aufkonzentriert wird.

9. Verfahren nach Anspruch 8,
dadurch gekennzeichnet,
daß abgetrenntes Cumol oder sek.-Butylbenzol oder beides zur Oxidation zurückgeführt wird.

10. Verfahren nach zumindest einem der vorherigen Ansprüche,
dadurch gekennzeichnet,
daß das Spaltgemisch homogen ist.

11. Verfahren nach zumindest einem der vorherigen Ansprüche,
dadurch gekennzeichnet,
daß die Spaltung säurekatalysiert wird.

12. Verfahren nach Anspruch 11,
dadurch gekennzeichnet,
daß als Säure Schwefelsäure verwendet wird.
